## Europäisches Patentamt

## European Patent Office

## Office européen des brevets

(11) Veröffentlichungsnummer: **0 125 687**

**A1**

# EUROPÄISCHE PATENTANMELDUNG

(21) Anmeldenummer: **84105492.7**

(22) Anmeldetag: **15.05.84**

(51) Int. Cl.³: **A 61 B 1/26**

(30) Priorität: **17.05.83 DE 3317831**
**17.05.83 DE 3317832**

(43) Veröffentlichungstag der Anmeldung:
**21.11.84 Patentblatt 84/47**

(84) Benannte Vertragsstaaten:
**AT BE CH FR GB IT LI LU NL**

(71) Anmelder: **GUSTAV MÜLLER GmbH & Co., KG. Fabrik für chirurgische Instrumente**
**Alleenstrasse 9**
**D-7200 Tuttlingen(DE)**

(72) Erfinder: **Müller, Rolf**
**Alleenstrasse 9**
**D-7200 Tuttlingen(DE)**

(74) Vertreter: **Bierl, Richard, Dr. rer. nat., Dipl.-Phys.**
**Hauptstrasse 32/I**
**D-7218 Trossingen 1(DE)**

(54) **Gerät für medizinische visuelle Untersuchungen von Körperhöhlen oder -gängen mit einem Handgriff.**

(57) Bei einem Gerät für medizinische visuelle Untersuchungen von Körperhöhlen oder -gängen mit einem Handgriff, in dessen rohrförmigem Gehäuse die Energiequelle für eine elektrische Lampe usw., der weiterhin eine Halterungsvorrichtung für ein schwenkbares Formteil mit Lichtkanal und die Lampe, sowie eine Welle für die Schwenkvorrichtung enthält, besteht die Neuerung darin, daß das rohrförmige Gehäuse an seinem einen Ende mit der Halterungsvorrichtung lösbar verschraubt ist, seitliche Lagerbacken für elektrisch leitende Verbindung mit einem Sockel und unterhalb desselben eine elektrisch isolierende Hülse enthält, die in der Bohrung für die Hülse schnappend und axial verschieblich steckbar gehalten ist, so daß die Lampe leicht ausgetauscht werden kann, und eine zweite metallische Hülse für eine mechanisch zweckmäßige und elektrisch gut leitende Verbindung mit der Lampenfassung für ihre Stromversorgung aus der Energiequelle enthält.

Die weitere Besonderheit in diesem Zusammenhang besteht darin, daß das gegenseitige Ende des rohrförmigen Gehäuses unter Federvorspannung durch einen Haubendeckel verschlossen ist, der bei Schwenkung des Formteils die Hülse mit der Lampe gegen die Anschlußkontakte drückt.d9.

FIG. 3b

**DIPLOMPHYSIKER DR. RER. NAT. RICHARD BIERL**

PATENTANWALTSBÜRO · SONNENWEG 2 · 7218 TROSSINGEN 1
ab 01.07.83 neue
Kanzleianschrift
Hauptstraße 32
Telex: 760 741 bipat D

PATENTANWALT
beim Deutschen Patentamt
Bundespatentgericht
8000 München 2

VERTRETER
beim Europäischen Patentamt
D-8000 München

0125687

Akt.-Zch.: P 33 17 831.3-35
"Laryngoskop"
Fa.Gustav Müller GmbH.&Co.KG.
72oo Tuttlingen

Beschreibung
Seite 1

13 o2o1/1 / Dr.Bi/bp
o7.Mai 1984

GUSTAV MÜLLER GmbH & Co., KG.
Fabrik für chirurgische Instrumente

72oo    T U T T L I N G E N

Gerät für medizinische visuelle Untersuchungen
von Körperhöhlen oder -gängen mit einem Handgriff

Die Erfindung betrifft ein Gerät für medizinische
visuelle Untersuchungen von Körperhöhlen- oder -gängen und
im besonderen ein Laryngoskop mit einem Handgriff und einem
hierfür üblichen Spatel mit seiner typischen Krümmung und
Verjüngung sowohl in der Breite als auch in der Höhe seines
Profils, sowie einem Handgriff, der in einem rohrförmigen
Gehäuse die Energiequelle für die Stromversorgung einer
elektrischen Lichtquelle, die elektrische Lichtquelle zB.
eine Glühlampe, mit Linsenkopf, sowie eine Halterungsvorrichtung mit einer Welle für ein Formteil enthält, das einen

- 2 -

Akt.-Zch.: P 33 17 831.3-35
"Laryngoskop"
Fa.Gustav Müller GmbH.&Co.KG.
7oo Tuttlingen

Beschreibung
Seite 2
13 o2o1/1 Dr.Bl/bp
o7.Mai 1984

0125687

Lichtkanal zwischen der Lichtquelle und einer entgegengesetzt zu letzterer liegender Lichtaustrittsöffnung enthält,
und auf die Welle schnappend aufsteckbar und um diese in
einer das rohrförmige Gehäuse enthaltenden Ebene in die
Arbeitslage rastend schwenkbar ist.

Die Einzelheiten des Erfindungsgegenstandes beziehen sich auf das in dem Handgriff enthaltene Gehäuse mit
einer Energiequelle und einer von dieser gespeisten elektrischen Lichtquelle und im besonderen auf eine Halterungsvorrichtung für ein in dieses aufsteckbares Formteil mit
Lichtkanal, dessen Schwenkbarkeit und inbesondere auf die
Ausbildung dieses Formteils, im Falle eines Laryngoskops
eines Spatels.

In der US-PS 3.638,644 ist auch schon ein Laryngoskop beschrieben, bei dem die Lichtquelle zusammen mit
ihrer Energiequelle in dem Handgriff untergebracht ist;
der Lichtkanal ist hierbei lediglich durch ein gebogenes
transparentes Kunststoffprofilstück gebildet; durch hochglänzend gemachte Innenwandungen und ein zusätzliches Ablenkblech werden Lichtverluste teilweise vermieden.

Bei diesem Gerät ist aber die Glühlampe, wenn sie
ausgetauscht werden muß, erst nach Entfernung des entgegengesetzt liegenden Verschlusses, der eingesetzten Batterie,
einer Federhalterung für eine Hülse, in der die Lampe eingesetzt ist, zur Entnahme erreichbar. Im übrigen weist das bekannte Gerät den Nachteil auf, daß die Lampe auch ohne Schwenkung des Spatels an die Energiequelle angeschlossen werden
kann. Normalerweise wird durch Schwenkung des Spatels in
die Arbeitslage der Hülsenfortsatz, dessen Randabsatz als
Anschlag gegen Herausfallen dient, beaufschlagt und damit
die Hülse zusammen mit dem Mittelanschluß der Lampe auf den
Mittelkontakt der Lampe gedrückt und so der Stromkreis von

- 3 -

Akt.-Zch.: P 33 17 831.3-35
"Laryngoskop"
Fa.Gustav Müller GmbH.&Co.KG.
7oo Tuttlingen

Beschreibung
Seite 3
13 o2o1/012-56/87
07.Mai 1984

Lampe und Energiequelle geschlossen; wie der Kontakt mit dem anderen Pol der Batterie hergestellt wird, ist nicht verständlich beschrieben bzw. der Druckschrift nicht zu entnehmen.

Auf jeden Fall hat diese bekannte Vorrichtung zunächst den schwerwiegenden Nachteil, daß es mehrerer sehr umständlicher Handgriffe bedarf, um die Lampe austauschen zu können, was zB. im Falle einer Operation od.dgl. mit großen Gefahren für den Patienten verbunden ist. Außerdem besteht die Gefahr, daß zufällig, zB. durch Klemmen der Hülse oder des Hülsenfortsatzes in der Handgriff-Kopfteil-Bohrung oder durch manuelles Niederdrücken des vorstehenden Hülsenfortsatzes die Lampe ungewollt oder versehentlich dauernd brennt, wobei die Batterie vorzeitig entladen wird, so daß das Gerät im Einsatzfalle nicht ausreichend funktionsfähig ist.

Die bekannten Geräte haben entweder den Nachteil, daß sie aus ziemlich vielen Teilen zusammengesetzt sind, und zwar so, daß es schwierig ist, die Oberfläche der mit Körperhöhlen-Wandungen in Berührung kommenden Flächen extrem glatt auszubilden, oder den anderen Nachteil, daß die Lichtleitung extrem lang und/oder mit erheblichen Lichtverlusten behaftet ist. Der vorliegenden Erfindung liegt deshalb die Aufgabe zugrunde, diese Nachteile zu vermeiden und zusätzlich, die Geräte so zu verbessern, daß die mit Wandungen von Körperhöhlen- oder -gängen in Berührung kommenden Oberflächen keine Werkstofflücken ausweisen können, die als Schlupfwinkel von Bakterien dienen und wegen ihrer außerordentlich geringen Größe nicht absolut einwandfrei gereinigt bzw. desinfiziert werden können. Außerdem soll das Gerät aus weniger und einfacheren Teilen zusammengesetzt und somit gegenüber den bekannten so verbessert und vereinfacht sein, daß unvermeidbare Austausch-Maßnahmen wesentlich

Akt.-Zch.: P 33 17 831.3-35
"Laryngoskop"
Fa.Gustav Müller GmbH.&Co.KG.
72oo Tuttlingen

Beschreibung 0125687
Seite 4
13 o2o1/1 / Dr.Bi/bp
o7.Mai 1984

schneller und einfacher ausgeführt werden können als bei den bekannten Geräten des Standes der Technik, so daß Komplikationen bei der Verwendung in der medizinischen Praxis, dh. während der ärztlichen Behandlung eines Patienten und damit auch Gefahren von gesundheitlichen Schäden für ihn, die auf Mängel an chirurgischen Instrumenten zurückgeführt werden müßten, völlig ausgeschlossen werden können.

Diese Aufgabe wird erfindungsgemäß durch die Ausbildung des Gegenstandes laut dem Oberbegriff des 1.Anspruchs durch Kombination mit den Merkmalen gemäß dem kennzeichnenden Teil des 1.Anspruchs gelöst. Besonderheiten für die Anwendung dieser Lösung auf ein Laryngoskop sind Gegenstand des 3.Anspruchs. Die Lichtquelle kann dabei im übrigen in einfacher Weise durch Abschrauben eines Kopfteils des Handgriffs entnommen und ausgetauscht werden und außerdem wird sie mit der Energiequelle elektrisch nur in der Betriebslage des Formteils mit Strom versorgt. Der Handgriff besteht dabei aus so wenig Teilen, daß seine Herstellung in der Serie besonders kostengünstig ist.

Weiterbildungen und ergänzende Besonderheiten für die grundsätzliche Lösung gemäß Anspruch 1 sind Gegenstände der Unteransprüche, wie auch die Anpassung an den Spezialfall des eigentlichen Laryngoskops. Im folgenden sind Ausführungsbeispiele anhand der Zeichnungen beschrieben; es stellen dar:

Fig.1: zwecks Vorauserläuterung der üblichen Teile und ihrer Bezeichnungen das Laryngoskop gemäß US-PS 3,638,644

a) in der zur Aufbewahrung bestimmten Lage mit abgeschwenktem Spatel

Akt.-Zch.: P 33 17 831.3-35
"Laryngoskop"
Fa.Gustav Müller GmbH.&Co.KG.
72oo Tuttlingen

Beschr.0125687
Seite
13 o2o1/1 /
o7.Mai 1984

b) in der Betriebslage

c) in einem Schnitt mit in die Betriebslage geschwenktem Spatel

Fig.2: in perspektivischer Darstellung

a) einen Spatel im nahezu kompletten
Zustand, lediglich ohne Lichtleiter

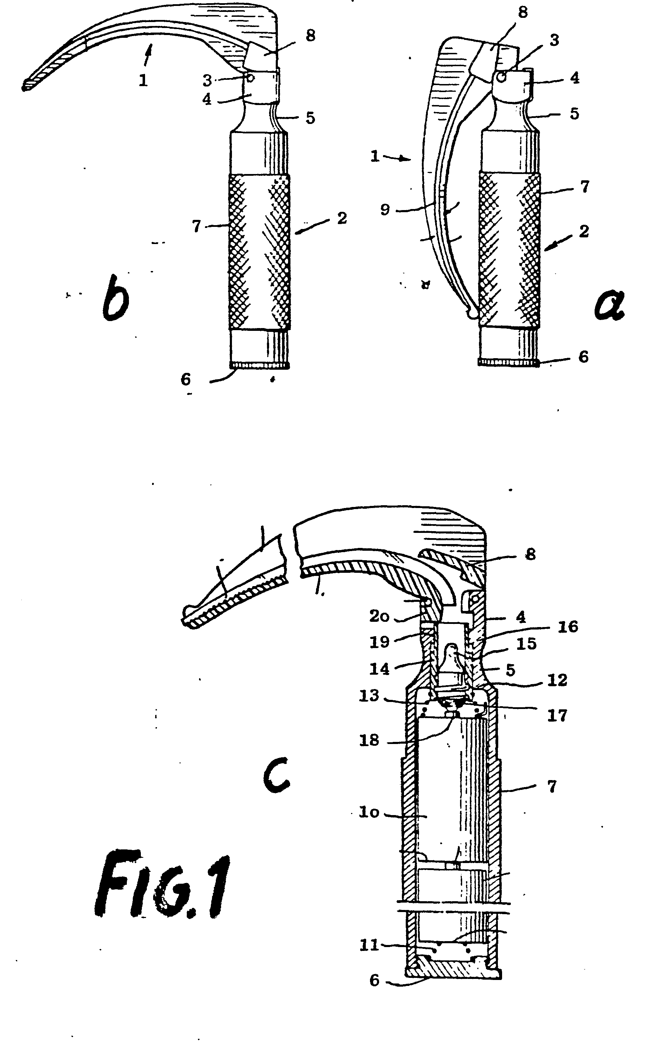

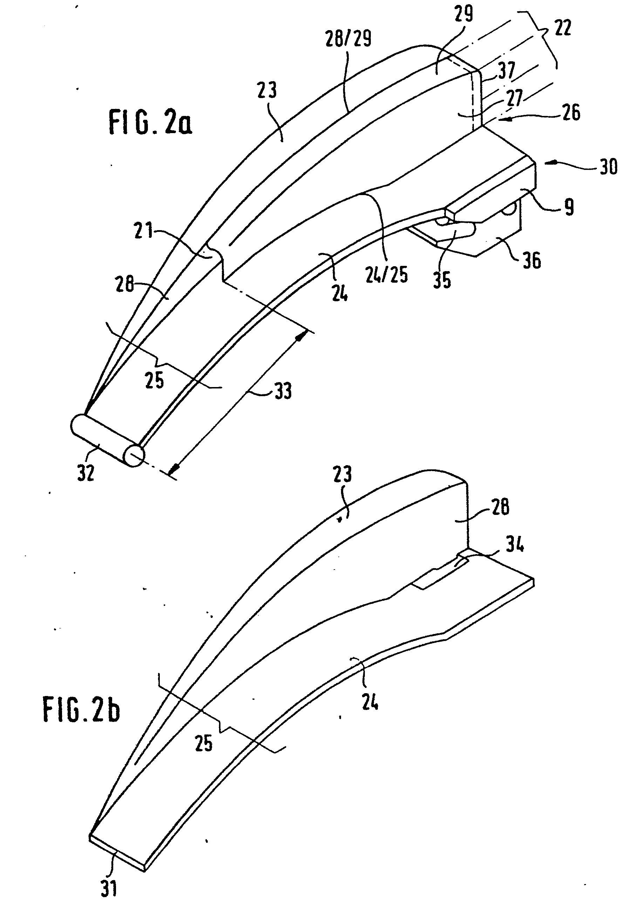

b) das Z-Profilstück als Ausgangsteil
des Spatels von Fig. 2a

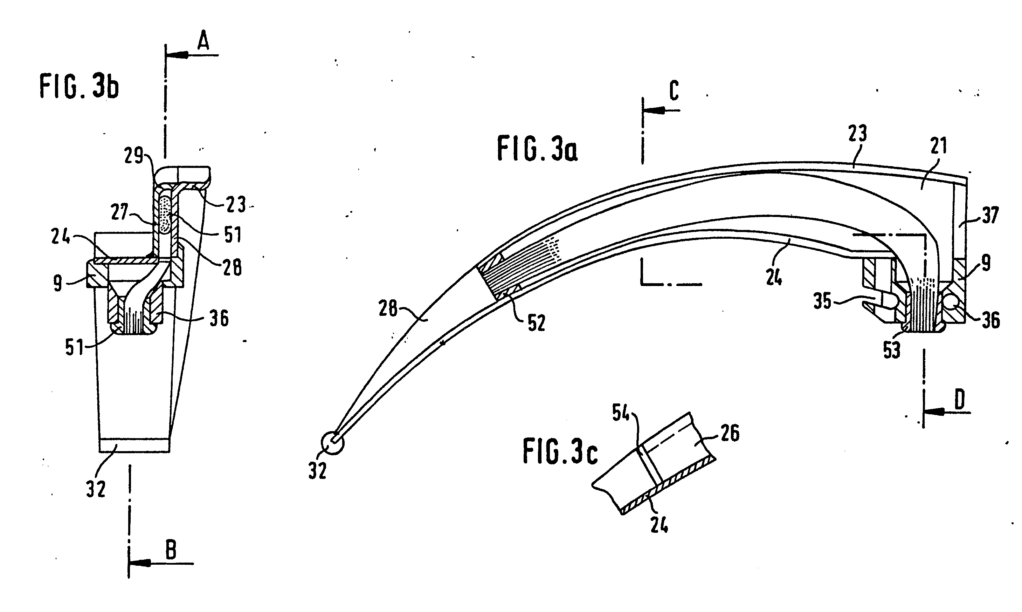

Fig.3: jeweils teilweise versetzt

a) einen Längsschnitt in den Ebenen
A und B der Fig. 3b

b) einen Querschnitt in den Ebenen
C und D, sowie in der Rückansicht
(vom proximalen Ende her)

c) eine Seitenansicht des Details
einer Hülse für den Glasfiber-
Lichtleiter

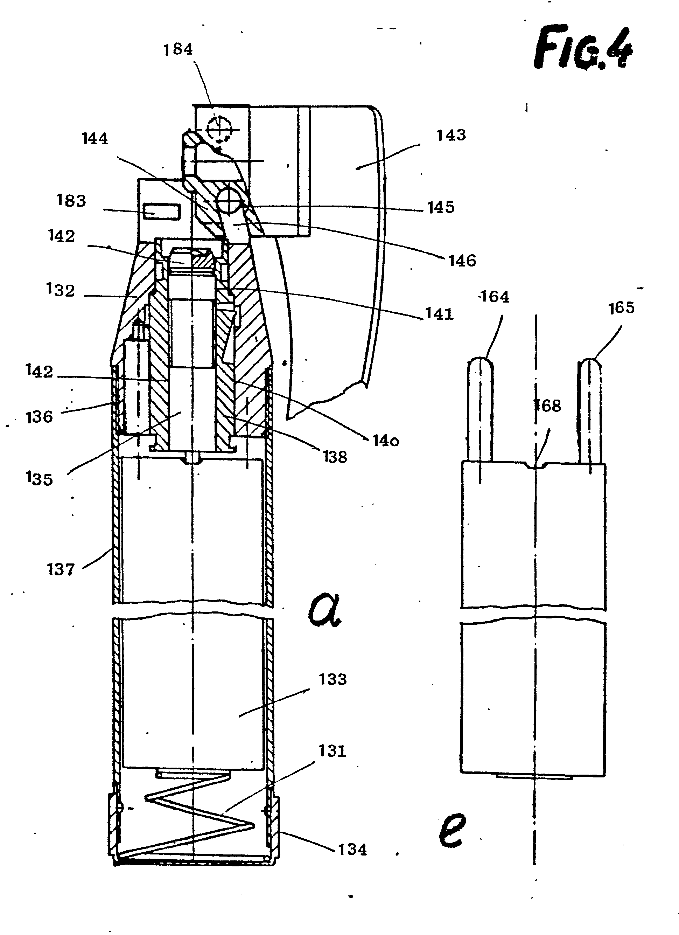

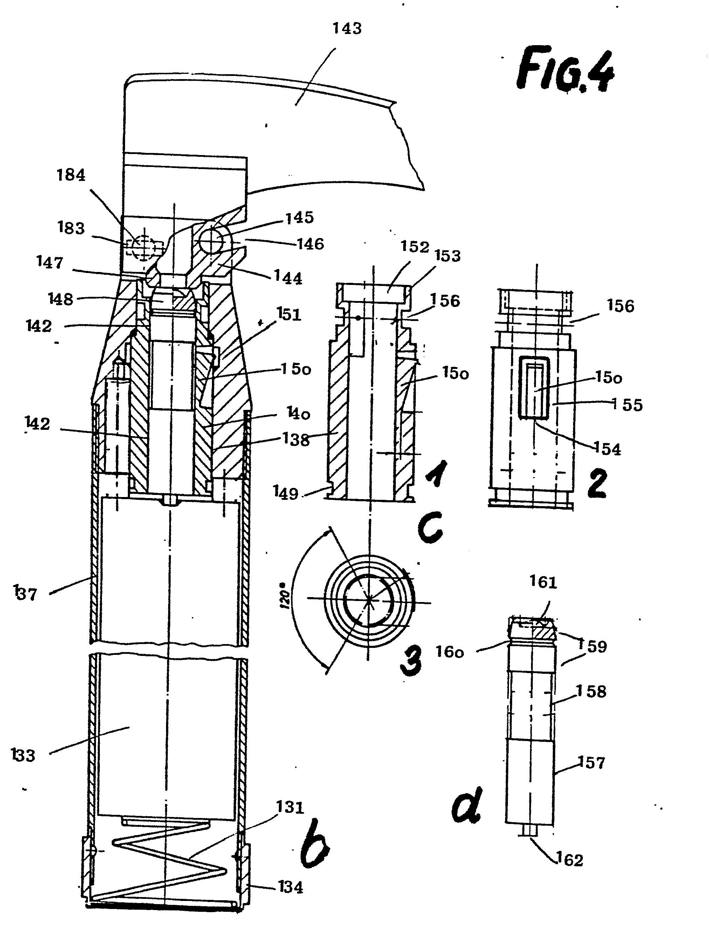

Fig.4: ein Ausführungsbeispiel des Geräts gemäß
Anspruch 1 (mit eingesetzter Batterie
als Energiequelle)

a) im (teilweise abgebrochen) Schnitt
des Handgriffs und teilweise abgebrochen und teilweise geschnitten
gezeichneter Darstellung des Formteils bei Außerbetriebslage

b) in der Betriebslage

c) die erste zylindrische Hülse aus
isolierendem Werkstoff bzw. Kunststoff

1. im Schnitt

2. in der Ansicht auf die Schnappfederseite

3. im Grundriß

d) die zweite, metallische Hülse mit eingebauter Lichtquelle und Linsenkopf

e) einen hierfür anstelle der Batterie
geeigneten Akkumulator-Einsatz

Akt.-Zch.: P 33 17 831.3-35
"Laryngoskop"
Fa.Gustav Müller GmbH.&Co.KG.
7200 Tuttlingen

Beschreibung
Seite 0125687
13 o2o1/1 / Dr.Bi/bp
o7.Mai 1984

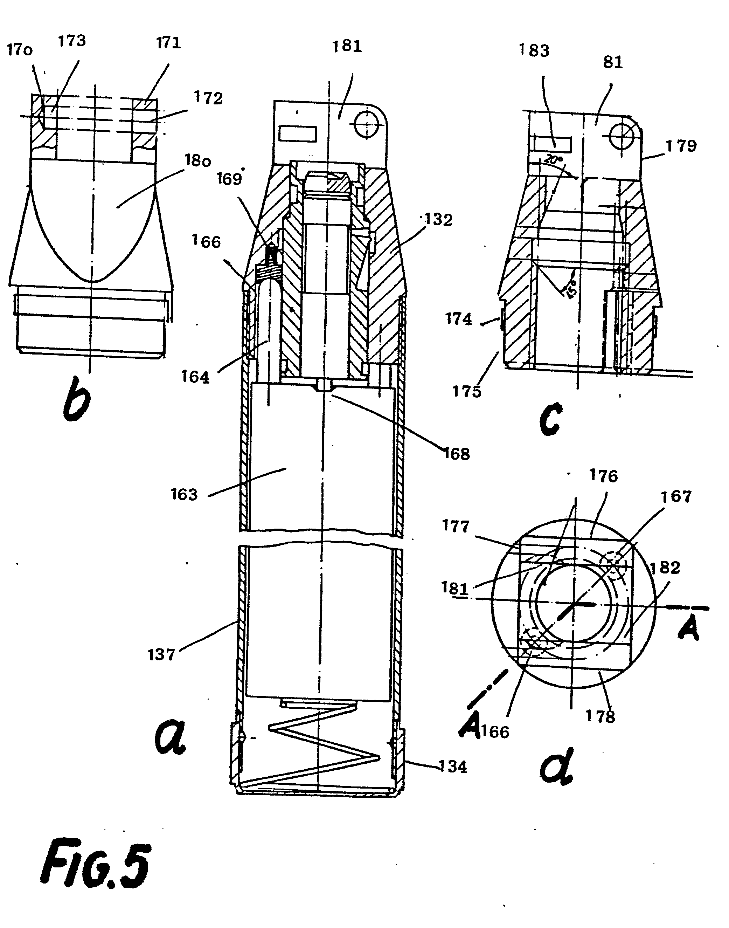

Fig.5: die Besonderheiten des Kopfsteils der
Halterungsvorrichtung

a) mit einem in den Handgriff eingesetzten Akkumulator-Einsatz und im Schnitt
entlang der Schnittlinie A-A (vgl.
Fig.5d)

b) in der Seitenansicht

c) im Schnitt entlang der Schnittlinie
A-A (vgl.Fig.5d) der Kopfteil allein

d) in der Draufsicht

Fig. 1a und b zeigen das Laryngoskop gemäß der US-PS 3,638,644 - a) in der zur Aufbewahrung bestimmten Lage mit abgeschwenktem Spatel 1 und b) in der Betriebslage - mit dem Handgriff 2, in dessen Gelenkstelle mit der Welle 3 in seinem Kopfteil 4 mit Gelenkteil 5 und dem Schraubverschluß des rohrförmig ausgebildeten Mittelteils 7. Der Spatel 1 weist ein Abschirmungsblech 8 zur Abdeckung des Lichtkanals 9 nach außen auf, weil der Lichtkanal bei dieser Ausbildung nicht ohne besondere Mühe passend zu dem Kunststoff-Lichtleiter gemacht werden kann.

In dem rohrförmig ausgebildeten Mittelteil 7 ist die als Energiequelle dienende Batterie 1o eingesetzt, die durch die Feder 11 an die entgegengesetzte Endfläche 12 unter Zwischenlage der Feder 13 angedrückt wird. In der Betriebslage wird die Hülse 14, in welcher die Lampe 15 mit ihrem als der eine Anschluß dienenden Gehäuse eingeschraubt und die in der Außerbetriebslage durch die Federn 11 und 13 gegen den Absatzanschlag 16 angedrückt, so daß sich der Mittelkontakt 17 der Lampe 15 nicht in elektrischem Kontakt mit dem Mittelkontakt 18 der Batterie 1o befindet.

Akt.-Zch.: P 33 17 831.3-35
"Laryngoskop"
Fa.Gustav Müller GmbH.&Co.KG.
72oo Tuttlingen

**0125687**
Beschreibung
Seite 7
13 o2o1/1 / Dr.Bi/bp
o7.Mai 1984

Fig.2 zeigt ein Ausführungsbeispiel des Formteils in der erfindungsgemäßen Ausbildung mit Z-ähnlich förmigen Querschnitt und dem den Lichtkanal 21 bildenden doppelwandigen Mittelsteg 22, mit an dessen Seitenrändern Schenkeln, dem ersten 23 und dem zweiten 24 entgegengesetzt dazu rechtwinklig abgebogen, von denen im Falle des beispielsweise dargestellten Spatels der erstere als Zungen-Niederhalter und der zweite 24 als Gaumenstütze dient. Der doppelwandige Mittelsteg 22 ist dadurch gebildet, daß auf dem Z-Profilstück 25 das L-Profilstück 26 so angebracht ist, daß dessen längerer Schenkel 27 parallel zu der Mittelstegwand 28 im Abstand des kürzeren Schenkels 29 des L-Profilstücks 26 liegt; der dadurch gebildete Hohlraum für den Lichtkanal 21, dessen Ende zwischen dem proximalen 3o und dem distalen Ende 31 des Formstücks bzw. Spatels 1 um mindestens ein Drittel 33 der Gesamtlänge versetzt liegt, ist für die Aufnahme eines Lichtleiters, zB. aus Glasfiberfäden bestimmt.

Für das jenseitige Ende dieses Lichtleiters ist im ersten Schenkel 24 der Durchbruch 34 vorgesehen, wo sich im kompletten Zustand der Sockel 9 befindet, der den Schlitz 35 aufweist, auf bzw. in den eine nichtgezeichnete Welle eingesteckt wird, so daß der Sockel darauf schwenkbar gelagert ist. Der Sockel 9, bzw. das sockelartige Lagerstück ist mindestens annähernd bündig mit dem zweiten Schenkel 24 verbunden und weist an seiner entgegengesetzten Seite den Nocken 36 auf, der im rechten Winkel zu der Lagerstelle im Schlitz 35 eine Bohrung für den Lichtleiter enthält. An der Rückseite des Lichtkanals 21 zwischen Mittelsteg 28 und langem Schenkel 27 - bündig mit der Endkante derselben - ist als

Akt.-Zch.: P 33 17 831.3-35
"Laryngoskop"
Fa.Gustav Müller GmbH.&Co.KG.
72oo Tuttlingen

Beschreibung
Se 0125687
13 o2o1/1 / Dr.Bi/bp
07.Mai 1984

Verschluß der Deckel 37 eingelassen.

Die Verbindung 28/29 zwischen dem ersten Schenkel 23 des Z-Profilstücks 25 und der Mittelstegwand 28 mit dem kürzeren Schenkel 23 des L-Profilstücks ist als Schweißnaht, zB. gemäß einem Verfahren der elektrischen Lichtbogenschweißung unter Schutzgas ausgeführt, die anschließend feingeschliffen und feinpoliert wird, so daß die Stoßkante dieser Verbindung 28/29 oberflächen-homogen lückenfrei verschlossen ist, dh. weder Feinrisse noch Porennarben aufweisen kann, in denen sich Bakterien, der Reinigung bzw. Desinfizierung nicht zugänglich, ablagern könnten, wenn solche Lücken nicht durch die neue Art der Verbindung ausgeschlossen wären. Die Verbindungsstelle 24/25 des zweiten Schenkels 24 des Z-Profilstücks 25 mit dem längeren Schenkel 27 des L-Profilstücks 26 ist gemäß einem herkömmlichen Lötverfahren mit so viel Lötgut hergestellt, daß ein deutlich erkennbarer Abrundungsradius entsteht und somit auch dort eine weitgehend lückenfreie Oberfläche gewährleistet ist.

Die Details der Konstruktion eines gemäß den vorbeschriebenen Besonderheiten ausgebildeten Spatels mit erstem Schenkel 23, zweitem Schenkel 24 und Mittelstegwand 28 des Z-Profilstücks 25, längerem 27 und kürzerem Schenkel 29 des L-Profilstücks 26, dem im Mittelsteg 22 eingeschlossenen Lichtkanal 21 mit Glasfiber-Lichtleiter 51, dem eingelassenen Deckel 37, dem Sockel 9, der annähernd bündig mit dem Mittelsteg 22 usw. verbunden ist, dem Nokken 36 mit dem Schlitz 35 für seine Schwenklagerung, den Hülsen 52, 53 für die bündige Halterung des Glasfiber-Lichtleiters sind in Fig. 3 mit Hilfe von Schnittdar-

Akt.-Zch.: P 33 17 831.3-35
"Laryngoskop"
Fa.Gustav Müller GmbH.&Co.KG.
7oo Tuttlingen

stellungen längs mehrfach versetzter Schnittlinien A-B,
C-C bzw. D-D und der Rückansicht wiedergegeben.

Zu der Hülse 52 ist noch ergänzend zu bemerken, daß sie mit dem Deckel 53 ausgestattet ist, der eine Öffnung für den Glasfiber-Lichtleiter aufweist, in der er bündig gefaßt ist, und bündig mit den Schenkeln 27, 29 des L-Profilstücks 26 abschließt. Die Kanten des Deckels 53 können vor allem in den Ebenen der Flächen der Schenkel 27, 29 des L-Profilstücks wesentlich glatter verarbeitet und bündig mit den Schenkels 27, 29 gemacht werden, zB. durch Schleifen und Polieren, als eine Hülse, die in den Lichtkanal 21 eingesteckt wird und in der Fläche der Lichtaustrittsöffnung Stoßkanten enthält, die nur schlecht bearbeitet werden können. Deshalb verhindert die neue Art der Abdeckung auch ein Eindringen von Flüssigkeit in den Lichtkanal 21 besser als die bekannte. Die Einzelheiten dieser Ausbildung der Hülse 52 sind in der Teilansicht der Fig.3c wiedergegeben, in der der Deckel 54 seitlich sichtbar ist, wie er auf dem Ende des L-Profilstücks 26 mit bündigen Außenkanten aufliegt.

Bei dem Ausführungsbeispiel der Fig.4 gemäß dem Anspruch 1 ist zwar ebenfalls die Energiequelle, hier die mit der Feder 131 zum Kopfteil 132 hin vorgespannte Batterie 133 durch Öffnen des Bajonettverschlusses 134 - anstelle der bedienungsunfreundlicheren Verschraubung - und Entnahme der Feder 131 zu entnehmen und auszutauschen, wesentlich vereinfacht gegenüber dem bekannten Gerät ist aber der Austausch der Lichtquelleneinheit 135, indem das mit dem Ende des rohrförmigen Gehäuses 137 durch Feingewinde verschraubte Kopfteil 132 abgenommen wird. Dann kann nämlich sofort die erste Hülse 138, die mit einem Absatz 139 an einem zugehörigen Innenanschlag

Akt.-Zch.: P 33 17 831.3-35
"Laryngoskop"
Fa.Gustav Müller GmbH.&Co.KG.
72oo Tuttlingen

Beschreibung
Seite 1o
13 o2o1 A / Dr.Bi/bp
07.Mai 1984

0125687

der Bohrung 14o des Kopfsteils 132 unter der über die Batterie 133 übertragenen Vorspannung der Feder 131 angedrückt wird, entnommen werden. In einer Bohrung 141 der ersten Hülse 138 ist die eigentliche Lichtquelle (nicht zeichnerisch dargestellt) mit ihrer metallischen Hülse 142 gefaßt, beispielsweise durch Klemmsitz oder eine Schnappvorrichtung, wobei der Gehäuseanschluß der Lichtquelle mit der metallischen Hülse elektrische verbunden ist und ihr anderer Anschluß an dem zur Batterie 133 zeigenden Ende isoliert gegen die metallische Hülse 142 vorragt.

Das Formteil 143 ist durch seine Sockelbakken 144 auf der Welle 145 schnappend mittels des Schlitzes 146 aufsteckbar und um die Welle 145 schwenkbar gelagert. Bei Schwenkung des Formteils 143 in die in Fig. 4b dargestellte Betriebslage beaufschlagt der Sockelnocken 147 den Oberteil 48 der metallischen Hülse 142, wobei diese zusammen mit der ersten Hülse 138 in der Bohrung 14o etwas versetzt und die Batterie 133 die Feder 131 etwas zusammendrückt. Dabei wird also der Stromkreis zwischen Lichtquelle und Energiequelle ausschließlich über den metallischen Sockelnocken 147 des Formteils geschlossen; ein manuelles Drücken auf die Lichtquelle bzw. ihre metallische Hülse, dh. deren Oberteil 148 hat dagegen keine Wirkung.

Die erste Hülse 138 weist an ihrem unteren Ende die Ringnut 149 auf, mit der sie aus der Bohrung 14o gezogen werden kann; gehalten wird sie durch die Schnappnase 5o, die in die Ringnut 151 der Bohrung 14o einschnappt. Am Kopfende weist die erste Hülse 138 die kreisförmige Eintiefung 152 auf, die durch den ringförmigen Vorsprung 153 gebildet wird, und in die der Sockelnocken 147 eintauchen kann, um den Kontakt mit der metallischen Hülse

Akt.-Zch.: P 33 17 831.3-35
"Laryngoskop"
Fa.Gustav Müller GmbH.&Co.KG.
7200 Tuttlingen

Beschreihung
Seite 11
13 o2o1 A 1 / Dr.Bi/bp
07.Mai 1984

142, bzw. ihrem Kopfteil 148 herzustellen. Die Schnappnase 15o ist als Zunge ausgebildet, die an einem Ende 154 mit der Wandung der ersten Hülse 138 verbunden ist und sich in dem Ausschnitt 155 der Wandung frei bewegen kann. Durch die Ringnut 156 wird die elastische Nachgiebigkeit gegenüber Formabweichungen der metallischen Hülse 142 herbeigeführt, so daß die bruchempfindliche Lichtquelleneinheit 135 bei ihrer Befestigung in der ersten Hülse 138 nicht beschädigt werden kann.

Die metallische Hülse 142 ist aus einem unteren metallischen Rohrteil 157 mit Absatzstück 158 und dem Kopfteil 159, das durch eine weitere Ringnut 16o ebenfalls elastisch ausgebildet ist, zusammengesetzt, wobei der äußere Anschluß der nur mit ihrem Linsenkopf 161 sichtbaren eigentlichen Lichtquelle mit dem metallischen Rohrteil 157 mechanisch und elektrisch verbunden, der Glasteil in dem Kopfteil so gehalten sind, daß die Lichtquelle nicht herausfallen kann, und der andere Anschluß 162 der Lichtquelle den Boden des metallischen Rohrteils 157 isoliert durchragt. In einer besonderen Ausgestaltung können die metallische Hülse 142 einschließlich Lichtquelle und die erste Hülse 138 unlösbar miteinander als eine kompakte Austausch-Baugruppe vereinigt sein, die besonders einfach für den Ersatz gehandhabt werden kann.

In Fig.5 ist der Handgriff mit rohrförmigem Gehäuse 137, Bajonettverschluß 134 und dem als Halterungsteil dienenden Kopfteil 132 - bestückt mit einem Akkumulator-Einsatz 163 - dargestellt. Für diesen, dh. dessen Stiftanschlüsse 164, 165, vgl. auch Fig.4b, sind in dem Kopfteil die Sackbohrungen 166, 167 angebracht, von denen eine mit dem zugehörigen Stift 164 als der eine Pol der Energiequelle dient, während der andere Pol durch den Mittelanschluß 168

Akt.-Zch.: P 33 17 831.3-35
"Laryngoskop"
Fa.Gustav Müller GmbH.&Co.KG.
7200 Tuttlingen

Beschreibung
Seite 0125687
13 o2o1/T / Dr.Bl/bp
07.Mai 1984

gebildet wird. In den Sackbohrungen, zB. 166, liegen zwischen ihren Grundflächen und den Stiften, zB. 164, Federn 169 zur Verbesserung der Kontaktgabe. Die beiden Stiftanschlüsse 164, 165 dienen bekanntlich als Netzstecker, an dem einen von ihnen (164) und dem Mittelanschluß 168 liegen die Gleichspannungspole des Akkumulator-Einsatzes.

Fig.5 läßt auch die beiden Lagerbacken 17o, 171 des als Halterungsvorrichtung dienenden Kopfteils 132 mit den Bohrungen 172, 173 - letztere als Sackbohrung - für die nichtdargestellte Welle erkennen. An dem dem rohrförmigen Gehäuse 137 zugewandten Absatz ist eine Feingewindestufe 174 von kleinerer Höhe angeordnet, die die Verschraubung verbessert, während der verbleibende Abschnitt 175 zur leichteren Einführung in das rohrförmige Gehäuse 137 dient und am Ende bei der Stufe das Feingewinde hinterdreht ist, um es einwandfrei herstellen zu können. Die Außenflächen der Lagerbacken 17o, 171 - mit 176 bis 179 bezeichnet - sind als Anflächungen 80 des Kopfteils 132 fortgesetzt. In den Innenflächen 181, 182 sind Nuten, zB. 183, eingetieft, in die die mittels eingesetzter Feder vorgespannte Kugel 184 (vgl. Fig.4a und b) zur rastenden Halterung des Formteils 143 in der ausgeschwenkten Lage einschnappt.

**DIPLOMPHYSIKER DR. RER. NAT. RICHARD BIERL**

PATENTANWALTSBÜRO · SONNENWEG 2 · 7218 TROSSINGEN 1
ab 01.07.83 neue
Kanzleianschrift
Hauptstraße 32
Telex: 760 741 bipat D

**PATENTANWALT**
beim Deutschen Patentamt
Bundespatentgericht
8000 München 2

**VERTRETER**
beim Europäischen Patentamt
D-8000 München

Akt.-Zch.: P 33 17 831.3-35
"Laryngoskop"
Fa.Gustav Müller GmbH.&Co.KG.
7200 Tuttlingen

13 o2o1-1 / Dr.Bi/bp
o7. Mai 1984
Seite 13

P a t e n t a n s p r ü c h e

1. Gerät für medizinische visuelle Untersuchungen von Körperhöhlen oder -gängen mit einem Handgriff,

der in einem rohrförmigen Gehäuse die Energiequelle für die Stromversorgung einer elektrischen Lichtquelle, die elektrische Lichtquelle, zB. eine Glühlampe, mit Linsenkopf, sowie eine Halterungsvorrichtung für ein mit einem der Lichtquelle zugewandten Sockel, den Lichtkanal zwischen der Lichtquelle und einer dazu entgegengesetzt liegenden Lichtaustrittsöffnung ausgestattetes Formteil enthält,

das auf eine in der Halterungsvorrichtung angeordnete im rechten Winkel zu der Achse des Handgriffs eingesetzte Welle mit seinem einen Ende schnappend aufsteckbar und um diese in die Arbeitslage rastend schwenkbar gelagert

und von dieser bis zu seinem freien Ende verjüngt ist,

Akt.-Zch.: P 33 17 831.3-35
"Laryngoskop"
Fa.Gustav Müller GmbH.&Co.KG.
7200 Tuttlingen

13 o2o1-1 / Dr.Bi/bp
o7. Mai 1984 / Seite 14
0125687

das rohrförmige Gehäuse (137)

an seinem einen Ende mittels Feingewinde mit der Halterungsvorrichtung lösbar verschraubt und durch einen Haubendeckel verschließbar ist,

zwischen dem und der Energiequelle (133 bzw.163) eine Feder (131) vorgespannt eingesetzt und dadurch unter Zwischenlage der Energiequelle (133 bzw.163) die erste zylindrische Hülse (138) bis zum Anschlag an einem Absatz (139) im Kopfteil (132) der Halterungsvorrichtung angedrückt ist

die seitliche, mindestens teilweise metallische Lagerbacken (170,171) für die Welle (145) enthält, auf der der Sockel mit den Sockelbacken (144) elektrisch leitend gelagert ist,

so daß das Formteil (143) bei Schwenkung in seine Betriebslage mit seinem Sockelnocken (148) die zweite Hülse (142) mechanisch und elektrisch beaufschlagt und dadurch der Stromkreis von Lichtquelleneinheit (135) und Energiequelle (133 bzw.163) geschlossen wird,

dadurch gekennzeichnet, daß

(a) das Formteil (zB. der Spatel 1) mindestens in einem Teil seiner Länge von einem Z-ähnlich-förmigen Querschnitt mit dem den Lichtkanal (21) bildenden doppelwandigen Mittelsteg (22) in Verbindung mit an dessen Seitenrändern einem ersten im rechten Winkel dazu abgebogenen (23) und einem zweiten entgegengesetzt gebogenen Schenkel (24) ausgebildet ist,

Akt.-Zch.: P 33 17 831.3-35
"Laryngoskop"
Fa.Gustav Müller·GmbH.&Co.KG.
7200 Tuttlingen

13 o2o1·.1 / Dr.Bi/bp
o7. Mai 1984 / Seite 15
0125687

(b) indem das Formteil ein Z-Profilstück (25) mit durchgehend gleicher Wandstärke enthält,

(b₁) parallel zu dessen Mittelstegwand (28) an einer Seite und auf dem ersten Schenkel (23) des Z-Profilstücks (25) aufstoßend ein L-Profilstück (26) mit seinem längeren Schenkel (27) und mit seinem kürzeren (29) bündig mit dem zweiten Schenkel (24) des Z-Profilstücks (25) einstückig so formschlüssig angebracht ist,

(b₂) daß zwischen beiden, dh. der Mittelstegwand (28) und dem längeren Schenkel (27) des L-Profilstücks (26) der Lichtkanal (21) eingeschlossen und mindestens die äußere Stoßstelle (28/29) zwischen dem kürzeren Schenkel (29) und dem zweiten Schenkel (24) bzw. der Mittelstegwand (28) des Z-Profilstücks (25) durch oberflächen-homogen lückenfrei verschlossen einstückige Verbindung vereinigt sind.

2. Gerät nach Anspruch 1,

dadurch gekennzeichnet, daß der kürzere Schenkel (29) des L-Profilstücks (26) und der erste Schenkel (23) bzw. die Mittelstegwand (28) des Z-Profilstücks (25) nach einem Verfahren für elektrisches Lichtbogenschweißen unter Schutzg as und der längere Schenkel (27) des L-Profilstücks (26) mit dem zweiten Schenkel (24) des Z-Profilstücks (25) nach einem herkömmlichen Lötverfahren, jedoch mit so viel Lötgut verbunden sind, daß ein deutlich erkennbarer Abrundungsradius entsteht.

Akt.-Zch.: P 33 17 831.3-35
"Laryngoskop"
Fa.Gustav Müller GmbH.&Co.KG.
72oo Tuttlingen

13 o2o1-1 / Dr.Bi/bp
o7. Mai 1984 / Seite 16
0125687

3. Gerät nach einem der Ansprüche 1 und 2, für den Mund, zB. mit Zungen-Niederhalter, insbesondere Laryngoskop, dadurch gekennzeichnet, daß das Formteil ein bei Laryngoskopen typischer Spatel (1) mit der dafür üblichen Krümmung und konstanten Breite des als Gaumenstütze dienenden zweiten Schenkels (24) und Verjüngung sowohl des als Zungen-Niederhalter dienenden ersten Schenkels (23) vom proximalen (3o) bis zum distalen Ende (31) in der Breite als auch des Mittelstegs (22) bzw. der Mittelstegwand (28) in der Höhe des Z-Profilstücks (25) ist,

(a) dessen zweiter zum Doppelwandteil im rechten Winkel liegender Schenkel (24) an seinem Ende beidseitig mindestens annähernd bündig mit einem Sockel (9) verbunden ist, der die Schwenklagerstelle mit Schlitz (35) und einen mittigen Nocken (36) enthält,

($a_1$) der eine Hülse mit mittig im rechten Winkel zu der Schwenklagerstelle stehenden Achse für die unversetzbare Halterung des einen Endes eines Glasfiber-Lichtleiters (51)

($a_2$) und an der dem Lichtleiter (51) zugewandten Seite und dem unteren Ende eine Nut bzw. einen Schlitz (35) für die Aufnahme der Welle aufweist,

(b) wobei das Ende des aus L-Profilstück (26) und Mittelstegwand (28) und einem Teil des zweiten Schenkels (24) des Z-Profilstücks (25) gebildeten Lichtkanals (21) mit der Lichtaustrittsöffnung zwischen proxima-

Akt.-Zch.: P 33 17 831.3-35
"Laryngoskop"
Fa.Gustav Müller GmbH.&Co.KG.
7200 Tuttlingen

13 o2o1 01/25687 Dr.Bi/Bp
07. Mai 1984 / Seite 17

lem (3o) und distalem Ende (31) des Spatels mit dem dort bündig festgehaltenen Ende des Glasfiber-Lichtleiters (51) mindestens annähernd um ein Drittel (33) der Gesamtlänge' vom distalen Ende (31) des Formteils bzw. Spatels (1) versetzt ist und sich die oberflächen-homogen lückenfrei einstückige Verbindung (28/29) über die ganze Länge des Lichtkanal-Abschnitts erstreckt, sowie dieser an seinem entgegengesetzten proximalen Ende (3o) durch einen bündig in der Rückwand des Sockels (9) liegenden Deckel (37) abgeschlossen ist.

4. Gerät nach einem der Ansprüche 1 bis 3,

dadurch gekennzeichnet, daß der Glasfiber-Lichtleiter (51) an seinem Ende nahe dem distalen Ende (31) des Formteils bzw. des Spatels (1) durch eine Hülse (52) mit Abschlußrand unversetzbar gefaßt ist und die Hülse (52) in den Lichtkanal (21) eingesteckt, sowie der Abschlußrand bündig mit dem Ende des L-Profilstücks (26), gegebenenfalls durch Feinschleifen und/oder Polieren, bearbeitet sind.

5. Gerät nach einem der Ansprüche 1 bis 4,

dadurch gekennzeichnet, daß das rohrförmige Gehäuse (137)

(a) und unterhalb der Halterungsvorrichtung eine Bohrung (14o) für eine erste zylindrische Hülse (138) aus isolierendem Werkstoff, insbesondere Kunststoff aufweist,

- 18 -

Akt.-Zch.: P 33 17 831.3-35
"Laryngoskop"
Fa.Gustav Müller GmbH.&Co.KG.
7200 Tuttlingen

13 o2o1-1 / Dr.Bi/bp
07. Mai 1982
0125687
- 18

($a_{1.1}$) die in der Bohrung (140) schnappend und axial verschieblich steckbar gehalten ist,

($a_{1.2}$) und eine zweite, metallische Hülse (142) enthält

($a_{1.2.1}$) die die Lichtquelle mindestens teilweise umschließt,

($a_{1.2.2}$) mit dem einen Anschluß der Lichtquelle mechanisch und elektrisch leitend verbunden ist

($a_{1.2.3}$) und den Linsenkopf (161) der Lichtquelle überragt,

($a_{1.3}$) und an deren unterem der Energiequelle (Batterie 133 oder Akkumulator-Einsatz 163) zugewandten Ende der andere Anschluß (162) der Lichtquelle mit dem einen Pol der Energiequelle (133 bzw. 163) verbindbar vorragt,

(b) und der hierüber eine elektrisch leitende Verbindung zwischen dem anderen Pol der Energiequelle (133 bzw. 163) und dem rohrförmigen Gehäuse (137), dh. auch der Halterungsvorrichtung mit Lagerbacken (170, 171) und Welle (145) herstellt, so daß in der Betriebslage des Formteils (143) sein metallischer Sockelnocken (147) in dessen eingerasteter Endlage unter Druck der im Bereich des als Haubendeckel ausgebildeten Bajonettverschlusses (134) eingesetzten Feder (131) vorgespannten ersten Hülse (138) auf dem Kopfteil (159) der zweiten, metallischen Hülse (142) aufliegt.

- 19 -

Akt.-Zch.: P 33 17 831.3-35
"Laryngoskop"
Fa.Gustav Müller GmbH.&Co.KG.
7200 Tuttlingen

13 o2o1-1 / Dr.Pi/bp
o7. Mai 1984 / Seite 19
0125687

6. Gerät nach einem der Ansprüche 1 bis 5,

dadurch gekennzeichnet, daß die Bohrung (14o) der Halterungsvorrichtung bzw. ihres Kopfteils (132) eine Ringnut (151) aufweist, mit der eine federnde Längsnase (15o) der ersten Hülse (138) schnappend zusammenwirkt, wobei in der Höhe dieser Längsnase (15o) die metallische Hülse (142) einen Absatz (158) mit kleinerem Durchmesser besitzt.

7. Gerät nach einem der Ansprüche 5 und 6,

dadurch gekennzeichnet, daß

(a) die zweite, metallische Hülse (142) über das der Halterungsvorrichtung bzw. deren Kopfteil (132) zugewandte obere Ende der ersten Hülse (138) vorragt und letztere am Außenrand einen ringförmigen Vorsprung (153) aufweist

(b) und der Sockelnocken (147) des Formteils (143) an seinem der zweiten Hülse (142) zugewandten Ende einen kreisförmigen Vorsprung mit der Bohrung für den Lichtleiter mit einem Außendurchmesser aufweist, der kleiner ist als der Innendurchmesser des ringförmigen Vorsprungs (153) der ersten Hülse.

8. Gerät nach einem der Ansprüche 1 bis 7,

dadurch gekennzeichnet, daß die Halterungsvorrichtung in deren der Energiequelle (133 bzw. 163) zugewandtem Abschnitt des Kopfteils (132) zwei Sackbohrungen (166,

- 2o -

Akt.-Zch.: P 33 17 831.3-35
"Laryngoskop"
Fa.Gustav Müller GmbH.&Co.KG.
72oo Tuttlingen

13 o2o1-1 / Dr.Bi/bp
o7. Mai 1984 / Seite 2o
0125687

167) für die Netzanschlußstifte eines aus dem Wechselstromnetz nachladbaren, handelsüblichen Akkumulator-Einsatz es (163) aufweist, von denen einer (164) den einen Pol seines Gleichstromausgangs und der Mittelanschluß (168) oder das Gehäuse bzw. ein Teil davon den anderen Pol bilden.

9. Gerät nach einem der Ansprüche 5 bis 8,.

dadurch gekennzeichnet, daß die erste Hülse (138) mit der eingesetzten zweiten Hülse (142) und der Lichtquelle als kompakte Austausch-Baugruppe ausgebildet ist, in der die metallische Hülse (142) mit der Lichtquelle so eingegossen ist, daß der andere Anschluß (162) der Lichtquelle der Boden dieser Baugruppe - gegebenenfalls von der metallischen Hülse (157) isoliert - durch-ragt.

1o. Gerät nach einem der Ansprüche 1 bis 7, für den Mund, zB. mit Zungen-Niederhalter, insbesondere Laryngoskop,

dadurch gekennzeichnet, daß

(a) das Formteil (143) ein bei Laryngoskop üblicher Spatel mit der dafür typischen Krümmung und Verjüngung sowohl in der Breite als auch in der Höhe seines Profils ist, das Z-ähnlich ausgebildet mit einem über einen Teil seiner Länge doppelwandigen Zwischensteg und dazwischen liegendem Lichtkanal und zwei entgegengesetzt gerichtet abgewinkelten Schenkeln ist,

Akt.-Zch.: P 33 17 831.3-35
"Laryngoskop"
Fa.Gustav Müller GmbH.&Co.KG.
72oo Tuttlingen

13 o2o1-1 / Dr.Bi/bp
07. Mai 1984  2 5687

(b) und der metallische Sockel mit Sockelbacken (144) und Sockelnocken (147) des Formteils (143) rechtwinklig zu seiner Längserstreckung angeordneten, auf der Welle (145) gelagerten Sockelnocken (147) so einrastet, daß in der Betriebslage des Formteils (143) mit mindestens annähernd rechtem Winkel zu der Achse des Handgriffs bzw. seines rohrförmigen Gehäuses (137) die vorgespannte zweite Hülse (142) von dem Sockelnocken (147) mechanisch und elektrisch leitend beaufschlagt wird.

Laryngoskop-Handgriff
Fa. Gustav Muller GmbH & Co., KG.
13 o2o2/13 o2o2 a/15.Mai 1983

1/6

**FIG.1**

Dr. Richard Bier
Hauptstr. 32, Tel. 07425/6
7218 Trossingen

FIG. 2a

28/29    29    22
23                37
21                27    26
28                      30
24/25                   9
24    35    36
25    24/25
33
32

FIG. 2b

23
28
34

24
25
31

Aktenzeichen: P 33 17 831.3
PatAnm. "Laryngoskop-Spatel"
Fa. Gustav Müller GmbH & Co.KG., 7200 Tuttlingen

Aktenzeichen: P 33 17 831.3
PatAnm. "Laryngoskop-Spatel"
Fa. Gustav Müller GmbH & Co., KG., 72oo Tuttlingen

Dr. Richard Bie
Hauptstr. 32, Tel. 07425/
7218 Trossingen

FIG. 3b

FIG. 3a

FIG. 3c

0125687

3/6

Akt.-Zch.: P 33 17 831.3-35
"Laryngoskop"
FA. Gustav Müller GmbH.& Co.KG.
7200 Tuttlingen

0125687

FIG.4

a

e

Akt.-Zch.: P 33 17 831.3-35
"Laryngoskop"
FA. Gustav Müller GmbH.& Co.KG.
7200 Tuttlingen

5/6

0125687

**FIG.4**

Akt.-Zch.: P 33 17 831.3-35
"Laryngoskop"
FA. Gustav Müller GmbH.& Co.KG.
7200 Tuttlingen

6/6

0125687

**b**

**a**

**c**

**d**

# FIG.5

**0125687**
Nummer der Anmeldung

## EUROPÄISCHER RECHERCHENBERICHT

Europäisches Patentamt

EP 84 10 5492

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl ³) |
|---|---|---|---|
| Y | DE-A-2 738 202 (HEINE OPTOTECHNIK GmbH & CO.) * Seite 7, Zeile 10 - Seite 11, Zeile 29; Figuren 1-6 * | 1 | A 61 B 1/26 |
| A | | 5,8,10 | |
| | --- | | |
| Y | FR-A-2 505 644 (HEINE OPTOTECHNIK GmbH & CO.) * Seite 3, Zeile 8 - Seite 4, Zeile 5; Figuren 1-4 * | 1 | |
| A | | 2,3,4, 10 | |
| | --- | | |
| A | EP-A-0 030 014 (M.S. UPSHER) * Seite 9, Zeile 18 - Seite 24, Zeile 4; Figuren 1-54 * | 1,3,5, 10 | RECHERCHIERTE SACHGEBIETE (Int. Cl. ³) A 61 B |
| | --- | | |
| A | WO-A-8 301 373 (M.S. UPSHER) * Seite 6, Zeile 19 - Seite 21, Zeile 36; Figuren 1-23 * | 1,3,5, 10 | |
| | --- | | |
| A | FR-A-2 361 855 (L. LEPELLETIER) * Seite 3, Zeile 26 - Seite 6, Zeile 23; Figuren 1-2 * | 1 | |
| | --- -/- | | |

Der vorliegende Recherchenbericht wurde für alle Patentansprüche erstellt.

| Recherchenort DEN HAAG | Abschlußdatum der Recherche 23-08-1984 | Prüfer ZILLIOX J.M. |
|---|---|---|

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | DE-B-1 766 713 (MEDICON CHIRURGIEMECHENIKER-GENOSSENSCHAFT) <br> * Spalte 1, Zeile 43 - Spalte 3, Zeile 29; Figuren 1-4 * <br><br> --- | 1-3 | |
| D,A | US-A-3 638 644 (F.G. REICK) <br><br> * Insgesamt * <br><br> ----- | | |

| | | | RECHERCHIERTE SACHGEBIETE (Int. Cl. ³) |

Der vorliegende Recherchenbericht wurde für alle Patentansprüche erstellt.

| Recherchenort <br> DEN HAAG | Abschlußdatum der Recherche <br> 23-08-1984 | Prüfer <br> ZILLIOX J.M. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument